(19) Europäisches Patentamt
European Patent Office
Office européen des brevets

(11) **EP 3 955 988 B1**

(12) **EUROPÄISCHE PATENTSCHRIFT**

(45) Veröffentlichungstag und Bekanntmachung des Hinweises auf die Patenterteilung:
**06.03.2024 Patentblatt 2024/10**

(21) Anmeldenummer: **20721164.0**

(22) Anmeldetag: **14.04.2020**

(51) Internationale Patentklassifikation (IPC):
***A61M 1/16*** (2006.01)

(52) Gemeinsame Patentklassifikation (CPC):
**A61M 1/1652;** A61M 1/1609; A61M 1/165

(86) Internationale Anmeldenummer:
**PCT/EP2020/060435**

(87) Internationale Veröffentlichungsnummer:
**WO 2020/212332 (22.10.2020 Gazette 2020/43)**

(54) **REZIRKULATIONSMESSUNG MITTELS DIFFUSIONSGLEICHGEWICHT**

RECIRCULATION MEASUREMENT BY MEANS OF DIFFUSION EQUILIBRIUM

MESURE DE RECIRCULATION AU MOYEN D'UN ÉQUILIBRE DE DIFFUSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.04.2019 DE 102019110218**

(43) Veröffentlichungstag der Anmeldung:
**23.02.2022 Patentblatt 2022/08**

(73) Patentinhaber: **B. Braun Avitum AG**
**34212 Melsungen (DE)**

(72) Erfinder:
• **ALDAG, Nina**
**30177 Hannover (DE)**
• **JANIK, Waldemar**
**34212 Melsungen (DE)**
• **KRAUSE, Silvie**
**34212 Melsungen (DE)**
• **WOLFF, Henrik**
**34212 Melsungen-Adelshausen (DE)**

(74) Vertreter: **Winter, Brandl - Partnerschaft mbB**
**Alois-Steinecker-Straße 22**
**85354 Freising (DE)**

(56) Entgegenhaltungen:
**EP-A1- 2 783 715    EP-A1- 2 783 716**
**US-A- 5 849 179**

**Beschreibung**

Die Erfindung betrifft eine Vorrichtung zur Rezirkulationsmessung bei einer extrakorporalen Blutbehandlung, beispielsweise Hämodialyse, Hämofiltration und /oder Hämodiafiltration.

**Hintergrund der Erfindung**

Bei einer extrakorporalen Blutbehandlung, beispielsweise einer Blutreinigung in Form einer Hämodialyse, Hämofiltration oder Hämodiafiltration, wird einem Dialysepatienten Blut über einen arteriellen Gefäßzugang entnommen, in einem Dialysator behandelt und dem Patienten anschließend über einen venösen Gefäßzugang zurückgegeben. Bei chronisch kranken Patienten werden die extrakorporalen Blutbehandlungen so häufig vorgenommen, dass sich die Vene, über die das Blut nach der Behandlung zurückgegeben wird, auf Dauer entzünden und verkleben würde. Aus diesem Grund wird solchen Patienten operativ ein sogenannter Shunt angelegt, welcher eine Querverbindung zwischen der Arterie und der Vene des Patienten darstellt und als dauerhafte Punktionsstelle verwendet wird. In Folge des Shunts verdickt sich die Gefäßwand der Vene, sodass diese sich leichter punktieren lässt und damit einen einfacheren Zugang für die Dialyse ermöglicht. In den meisten Fällen wir ein solcher Shunt in den Arm eines Patienten integriert.

Über die Querverbindung zwischen Arterie und Vene kommt es jedoch auch zu einem Blutaustausch von venösem und arteriellem Blut, insbesondere wenn während der Blutbehandlung der Blutfluss an der Blutpumpe zu hoch eingestellt ist und infolgedessen das in den Shunt des Patienten zurückzufördernde Blut teilweise in den arteriellen Gefäßzugang des Patienten übertritt. Folglich verdünnt also das bereits gereinigte, venöse Blut das noch ungereinigte, arterielle Blut, sodass es zu einer Beeinträchtigung der Blutbehandlungseffizienz bzw. des Wirkungsgrads der Blutbehandlung kommt. Dadurch wird die Behandlungszeit verlängert. Ein solcher Vorgang, bei dem das bereits gereinigte sowie in den Patienten zurückgeförderte Blut aus der Patientenvene über den Gefäßzugang, bspw. Shunt, in die Arterie einströmt und vor dort erneut in den extrakorporalen Blutkreislauf gelangt, wird als Rezirkulation bezeichnet und kann anhand unterschiedlicher Methoden qualitativ sowie quantitativ bestimmt werden. Eine quantitative Rezirkulationsmessung wird unter anderem zur Überwachung des Shuntzustands und der Überprüfung der Blutbehandlungseinstellungen, beispielsweise die Förderrate der Blutpumpe, eingesetzt.

**Stand der Technik**

Aus dem Stand der Technik sind mehrere unterschiedliche Methoden zur Bestimmung des Rezirkulationsanteils bekannt. Ein häufig angewandtes Verfahren sieht beispielsweise die Erzeugung eines definierten Temperaturbolus vor, welcher im venösen Blutzweig aufgegeben wird, mit anschließender Temperaturmessung im arteriellen Zweig, sodass basierend auf der aufgegebenen Temperaturdifferenz und der gemessenen Temperaturdifferenz mathematisch ein Rückschluss auf die Rezirkulationsquote gebildet werden kann. Ebenso ist es bekannt, anstelle der Temperatur einen anderen Indikator, beispielsweise eine bestimmte Stoffkonzentration oder die Leitfähigkeit, zu messen, der zuvor in Form eines (Indikator-) Bolus aufgegeben wurde. Auch kann der Bolus dialysierflüssigkeitsseitig stromauf des Dialysators aufgegeben werden und dialysierflüssigkeitsseitig stromab des Dialysators eine entsprechende Messung durchgeführt werden, aus der die Rezirkulation bestimmbar ist.

Aus der Patentschrift DE 197 02 441 C1 ist beispielsweise eine Vorrichtung und ein Verfahren zur Bestimmung der Rezirkulation bei einer extrakorporalen Blutbehandlung unter Einsatz eines Shunts bekannt, wobei eine Indikatorgröße, beispielsweise ein Konzentrationsbolus, im Dialysierflüssigkeitskreislauf stromauf des Dialysators erzeugt und die Konzentration eine definierte Zeitspanne später im Dialysierflüssigkeitskreislauf stromab des Dialysators beobachtet wird, um einen Rückschluss auf die Rezirkulation zu ziehen.

Auch in EP 2 783 715 A1 ist ein Verfahren zur Rezirkulationsmessung offenbart, bei dem durch blutseitige Boluszugabe und dialysierflüssigkeitsseitigem spektralphotometrischem Messen eine Rezirkulation bestimmbar ist.

Als weiteres Beispiel beschreibt die Patentanmeldung US 5,588,959 A eine Vorrichtung und ein Verfahren für eine Rezirkulationsmessung mittels Temperatur. Dabei wird das Blut am venösen Schlauchabschnitt gekühlt und die Temperatur des Bluts im arteriellen Schlauchabschnitt gemessen.

US 5,849,179 A offenbart ein Online-Echtzeit-Hämodialyse-Überwachungssystem für die Hämodialyse-Behandlung, welches die Rate und Menge eines Bestandteils quantifiziert, wie z.B. Harnstoff, der während der Hämodialyse-Behandlung entfernt wird, indem die Konzentrationen des Bestandteils als Funktion der Zeit im verbrauchten Dialysat-Abfluss aus einer Hämodialyse-Maschine gemessen werden. Eine Menge des verbrauchten Dialysats wird in regelmäßigen Abständen aus der Dialysatabflussleitung entnommen und untersucht. Ein Zeitprofil der Harnstoffkonzentration kann analysiert werden, um die Indizes für Harnstoffentfernung, KT/V, URR, SRI und normalisierte Proteinkatabolisierungsrate (nPCR) zu bestimmen. Das Hämodialyseüberwachungssystem kann vorzugsweise eine mit dem Blut äquilibrierte Dialysatprobe vor Beginn einer Hämodialysebehandlung gewinnen.

EP 2 783 715 A1 der vorliegenden Anmelderin offenbart ein Steuerungsverfahren einer Dialysemaschine zur Ermöglichung der dialysierflüssigkeitsseitigen Erfassung einer Rezirkulation in einem arteriovenösen Ein-/Auslass-Element, vorzugsweise Shunt, wobei die Dialysemaschine wenigstens einen Dialysierflüssigkeitseinlass sowie wenigstens einen Dialysierflüssigkeitsabfluss für eine ausgewählte Dialysierflüssigkeit und wenigstens eine Dialysierflüssigkeitspumpe hat, welche in fluidischer Verbindung mit wenigstens einem Dyalisator stehen, wobei ein Sensor in Dialysierflüssigkeits-Strömungsrichtung gesehen dem Dialysator nachgelagert ist, der eine Änderung eines physikalisch-chemischen Parameters der abfließenden Dialysierflüssigkeit erfasst. Dabei umfasst das Steuerverfahren die folgenden Schritte: Einstellen eines gewünschten ersten Blutflusswerts $BF_1$, bei dem sich eine Rezirkulations R größergleich 0 einstellt und der vom Sensor erfasste Parameter einen entsprechenden ersten Parameterwert $P_{D1}$ annimmt; Ändern des ersten Blutflusswerts $BF_1$ auf einen vorzugsweise kleineren zweiten Blutflusswert $BF_2$, wobei am Sensor ein neuer zweiter Parameter-Wert $P_{D2}$ entsprechend vorliegt und erfasst wird; Bestimmen der Rezirkulation R anhand des Änderungsverlaufs und/oder der Änderung vom ersten Parameterwert $P_{D1}$ zum zweiten Parameter-Wert $P_{D2}$.

Weiter offenbart die Patentanmeldung WO 96/08305 A1 ein Verfahren zur Rezirkulationsbestimmung bei extrakorporalen Blutbehandlungen, indem ein Indikator im venösen Blutzweig zugegeben wird und mittels eines Detektors am arteriellen Blutzweig ein dem Indikator zugeordneter Messwert erfasst und anhand der Verdünnungskurve eine Rezirkulationsrate berechnet wird.

Weiter sind Verfahren zur Rezirkulationsbestimmung bekannt, bei denen die Rezirkulation anhand der folgenden Gleichung berechnet wird:

$$R = \frac{1 - \frac{c_{DO} \cdot Q_D}{c_{sys} \cdot K_D}}{1 - \frac{c_{DO} \cdot Q_D}{c_{sys} \cdot K_D} + \frac{c_{DO} \cdot Q_D}{c_{sys} \cdot Q_B}}$$

Dabei gilt folgendes:

| R | Rezirkulationsrate | |
|---|---|---|
| cDO | Konzentration eines Stoffes am Dialysatausgang | Messwert (aktuell) bzw. messbar |
| csys | c_sys systemische Konzentration eines Blutanteilsstoffes, welche nicht rezirkulationsbehaftet ist. (Konzentration im Patienten) | Messwert/Peakwert |
| QD | Dialysierflüssigkeitsflussrate | Bekannt bzw. einstellbar |
| QB | Blutflussrate im extrakorporalen Blutschlauchsystem | Bekannt bzw. einstellbar |
| KD | theoretische Clearance des Dialysators | Funktion von (KOA, QB, QD) → also bekannt |
| KOA | dialysatorspezifisch | bekannt (Lookup-Tabelle), d.h. spezifischer Clearancekoeffizient, der abhängig ist vom aktuell eingesetzten Dialysator und verwendeter Dialysierflüssigkeit und der vorab (empirisch) ermittelbar ist |

Diese Formel lässt sich, wie folgt, ableiten:

Die Rezirkulationsrate *R* wird im Allgemeinen nach folgender Gleichung bestimmt:

$$R = \frac{1 - \frac{K_E}{K_D}}{1 - \frac{K_E}{K_D} + \frac{K_E}{Q_B}} \quad (1)$$

Mit

R Rezirkulationsrate (0...1 bzw. 0...100%)
$K_E$ effektive Clearance
$K_D$ theoretische Clearance des Dialysators
$Q_B$ Blutflussrate im extrakorporalen Blutschlauchsystem (bekannt, da an der Maschine einstellbar)
(vgl. DE 10 2013 103 221 A1, Abschnitt [0123])

Falls die Rezirkulation 0 ist, gilt $K_E = K_D$. Der Wert $K_E$ kann demnach rezirkulationsbehaftet sein.

Es gilt weiterhin folgender Zusammenhang:

$$c_{DO} \cdot Q_D = c_{BI} \cdot K_D = c_{sys} \cdot K_E \qquad (2)$$

Mit

$Q_D$ Dialysierflüssigkeitsflussrate (bekannt, da an der Maschine einstellbar)
$c_{DO}$ Konzentration eines Stoffes am Dialysatausgang
$c_{BI}$ Konzentration eines Blutanteilsstoffes im arteriellen Zweig des Blutschlauchsystems
$c_{sys}$ systemische Konzentration eines Blutanteilsstoffes, welche nicht rezirkulationsbehaftet ist. (Konzentration im Patienten)

Somit lässt sich die effektive Clearance durch Umstellen von Gleichung (2) bestimmen zu:

$$K_E = \frac{c_{DO} \cdot Q_D}{c_{sys}} \qquad (3)$$

Nach der Gleichung von Michaels (Michaels. "Operating Parameters and Performance Criteria for Hemodialyzers and other Membrane - Separation Devices". In: Trans Amer Soc Artif Intern Organs 12 (1966), 387-392.) kann $K_D$ bestimmt werden, wenn die Flussraten $Q_D$ und $Q_B$ sowie der dialysatorspezifische $K_0A$-Wert bekannt sind:

$$K_D = Q_B \left| \frac{\exp\left[\frac{K_0A}{Q_B}\left(1 - \frac{Q_B}{Q_D}\right)\right] - 1}{\exp\left[\frac{K_0A}{Q_B}\left(1 - \frac{Q_B}{Q_D}\right)\right] - \frac{Q_B}{Q_D}} \right| \qquad (4)$$

Der $K_0A$-Wert ist für die unterschiedlichen Dialysatoren bekannt und kann bspw. einer im Speicher der Dialysemaschine hinterlegten Lookup-Tabelle entnommen werden.
Einsetzen der Gleichungen (3) in (1) ergibt:

$$R = \frac{1 - \frac{c_{DO} \cdot Q_D}{c_{sys} \cdot K_D}}{1 - \frac{c_{DO} \cdot Q_D}{c_{sys} \cdot K_D} + \frac{c_{DO} \cdot Q_D}{c_{sys} \cdot Q_B}} \qquad (5)$$

Hierbei ist $K_D$ aus Gleichung (4) bekannt.
Die obigen Zusammenhänge gelten nur für den Fall, dass die Ultrafiltrationsrate zum Zeitpunkt der Messung minimal bzw. null gesetzt wird.
Bei den bekannten Verfahren, die diese Gleichung (3) zur Rezirkulationsberechnung heranziehen, wird deshalb

4

zur Bestimmung von $c_{sys}$ vor der Blutbehandlung zunächst eine Blutprobe des Patienten genommen und $c_{sys}$ gemessen. Anschließend kann die Rezirkulation R während der Blutbehandlung über die Gleichung (3) ausgerechnet werden.

Der Stand der Technik hat jedoch immer den Nachteil, dass entweder ein erhöhter apparativer Aufwand, beispielsweise durch Vorsehen von Temperiereinrichtungen oder Einrichtungen für eine Stoff-bzw. Konzentrationsboluszugabe, erforderlich ist oder vor der Behandlung eine separate Blutwertbestimmung durchgeführt werden muss. Des Weiteren ist die Bestimmung von $c_{sys}$ anhand einer Patientenblutprobe aufwändig und daher nicht zufriedenstellend.

**Kurze Beschreibung der Erfindung**

Es ist somit die Aufgabe der Erfindung, die Nachteile aus dem Stand der Technik zu überwinden oder zumindest zu mildern und insbesondere eine Vorrichtung zur Rezirkulationsmessung bei einer extrakorporalen Blutbehandlung, beispielsweise unter Verwendung eines Shunts, bereitzustellen, die ohne zusätzlichen apparativen Aufwand, beispielsweise einer Temperiereinrichtung, und/oder verfahrenstechnischen Aufwand, wie beispielweise einer separaten Blutprobenentnahme vor der Behandlung, durchführbar ist.

Die Aufgabe der Erfindung wird mit einer extrakorporalen Blutbehandlungsmaschine mit den Merkmalen des Anspruchs 1 gelöst.

Ein Grundgedanke der Erfindung besteht darin, eine Vorrichtung zur extrakorporalen Blutbehandlung zu schaffen, die dazu eingerichtet ist, die systemische Blutanteilstoff-Konzentration $c_{sys}$ (Konzentration eines Anteilsstoffs im Blut des Patientenkörpers) während der Blutbehandlung und insbesondere ausschließlich mit den an einer Vorrichtung zur extrakorporalen Blutbehandlung grundsätzlich vorgesehen Einrichtungen zu bestimmen und die Rezirkulation R dann anhand der vorstehenden, bekannten Gleichung (5) zu ermitteln. Anders ausgedrückt soll $c_{sys}$ bestimmt werden können, ohne dass dazu Apparaturen wie beispielsweise eine Temperiereinrichtung zur Erzeugung eines Temperaturbolus oder Einrichtungen zum Injizieren eines Stoffbolus benötigt werden.

Zu diesem Zweck bedient sich die Erfindung einer vorzugsweise optisch arbeitenden Sensorvorrichtung am oder stromab zu einem dialysierflüssigkeitsseitigen Dialysatorausgang, welche im Allgemeinen zur Erfassung/Bestimmung von bestimmten Blutstoffanteilen wie urämische Toxine (z.B. Harnstoff) in der verbrauchten Dialysierflüssigkeit verwendet werden sowie einer vorzugsweise Dialysemaschine-eigenen elektronischen Steuerung, welche erfindungsgemäß dafür vorgesehen und angepasst ist, die Dialysemaschine in einen Modus zu schalten, in welchem Dialysierflüssigkeit im Dialysator solange eingesperrt wird, bis sich auf der Dialysierflüssigkeitsseite und der Blutseite des Dialysators ein sich nicht mehr (oder unwesentlich) veränderndes Blutanteilstoff-Konzentrationsgleichgewicht einstellt/eingestellt hat. D.h., während der Einsperrphase nimmt die Reinigung des Blutes im Dialysator ab und geht gegen null, weshalb schließlich ein Diffusionsgleichgewicht zwischen Blut- und Dialysierflüssigkeitsseite herrscht. Rezirkulation spielt hierbei nur insoweit eine Rolle als dass sich die Zeitspanne bis zur Erreichung eines Diffusionsgleichgewichts verändert (verlängert).

Diese im Dialysator temporär eingesperrte Menge an verbrauchter Dialysierflüssigkeit hat demnach eine dem Patientenblut im Wesentlichen entsprechende Blutanteilstoff-Konzentration und kann dann wie ein Dialysierflüssigkeitsbolus der Sensorvorrichtung zugeführt werden, welche dessen Blutanteilstoff-Konzentration misst/erfasst. Der Wert $c_{sys}$ entspricht erfindungsgemäß dann dem Peak im Sensorsignal der Sensorvorrichtung (unmittelbar) nach der Freigabe des Dialysierflüssigkeitsbolus aus dem Dialysator.

An dieser Stelle sei darauf hingewiesen dass unter dem Begriff "im Dialysator eingesperrt" konkret ein Einsperren der Dialysierflüssigkeit auf einer Dialysierflüssigkeitsmembranseite des Dialysators zu verstehen ist. Der Begriff "einsperren" ist darüber hinaus als nicht bzw. im Wesentlichen nicht durchströmend zu verstehen. Weiter umfasst der eingeschlossene Raum u.U. auch noch Teile der Dialysierflüssigkeitszu-/ablaufleitung. Wenn man beispielsweise davon ausgehen würde, dass z.B. durch Aktivierung eines Dialysator-Bypasses mit einem deutlich geringeren Strömungswiderstand (im Vergleich mit dem Dialysator) ein Strömen der Dialysierflüssigkeit durch den Dialysator unterbrochen bzw. im Wesentlichen unterbrochen wird, könnte auf die dicht abschließende Ventile für eine solche Bypassschaltung verzichtet werden. In diesem Fall wäre die Flüssigkeit nicht wirklich ‚eingesperrt' sondern die Durchströmung des Dialysators mit Dialysierflüssigkeit wäre auf nahe Null reduziert.

Konkret ist erfindungsgemäß eine extrakorporale Blutbehandlungsmaschine, insbesondere Hämodialyse-, Hämofiltrations- oder Hämodiafiltrationsmaschine, vorgesehen (nachfolgend auch allgemein als Dialysemaschine bezeichnet) mit einem Dialysator, der einen Dialysierflüssigkeitszulauf für frische Dialysierflüssigkeit und einen Dialysierflüssigkeitsablauf für verbrauchte Dialysierflüssigkeit aufweist und der weiter mit einer Filtermembran ausgestattet ist, welche eine Dialysierflüssigkeitsmembranseite, auf der der Dialysator über eine Dialysierflüssigkeitszulaufleitung und eine Dialysierflüssigkeitsablaufleitung an einen Dialysierflüssigkeitskreis(lauf) angeschlossen ist, von einer Blutmembranseite trennt, auf der der Dialysator an einen extrakorporalen Blutkreis(lauf) angeschlossen oder anschließbar ist. Die erfindungsgemäße Dialysemaschine weist weiter bevorzugt eine Bypassleitung auf, mittels derer die Dialysierflüssigkeitsmembranseite wahlweise in einem Kurzschlussmodus überbrückbar ist, um im Dialysator befindliche Dialysierflüssigkeit temporär einzusperren. Hierzu ist an der Dialysierflüssigkeitszulaufleitung und der Dialysierflüssigkeitsablaufleitung wenigstens jeweils ein (Sperr)Ventil zwischen der Bypassleitung und dem Dialysator angeordnet. Weiter ist die erfindungsgemäße Dialysemaschine mit einer Sensorvorrichtung am oder stromab des Dialysierflüssigkeitsablaufs des Dialysators ausgestattet, die dazu angepasst ist, durch die Filtermembran hindurchgetretene Blutanteilsstoffe, insbesondere urämische Toxine (bspw. Harnstoff, Kreatinin, Harnsäure, Kalium etc.), in der aus dem Dialysator ablaufenden, verbrauchten Dialysierflüssigkeit messtechnisch, insbesondere optisch, zu erfassen. Generell ist an dieser Stelle anzumerken, dass mit dem Sensor eine physikalische oder chemische Größe gemessen wird, die zur Konzentration eines bestimmten Stoffes proportional sein kann. Tatsächliche bzw. unmittelbar gemessene Konzentrationswerte benötigt das vorgeschlagene Verfahren zur Rezirkulationsmessung nicht. Die erfindungsgemäße Dialysemaschine weist weiter eine Steuer- und Recheneinheit zur Steuerung der Dialysemaschine vorzugsweise mit einer Speichereinheit auf. Dabei ist die Dialysemaschine vorzugsweise weiter mit einem auf der Speichereinheit oder einem vergleichbaren separaten Speichermedium abgelegten oder ablegbaren Datensatz ausgestattet oder bestückbar, der zumindest für den aktuell angeschlossenen Dialysator einen Blutstromwert im extrakorporalen Blutkreis und einen zugehörigen, vorzugsweise analytisch bestimmten, Zeitwert umfasst, wobei der Blutstrom- und Zeitwert und/oder die Identität bzw. die Eigenschaften des angeschlossenen Dialysators natürlich auch manuell eingegeben, eingescannt oder auf andere Weise eingespeist werden könnten. Innerhalb dieses durch den Zeitwert bestimmten Zeitraums ist im Dialysator bei eingestelltem Blutstromwert unter der Annahme eines maximal möglichen Rezirkulationswerts (z.B. 20%) ein Konzentrationsausgleich zumindest eines ausgewählten oder auswählbaren Blutanteilsstoffs zwischen Blut im extrakorporalen Blutkreis und im Dialysator eingesperrter Dialysierflüssigkeit ausschließlich infolge von Diffusion beendet. Die Dialysemaschine verfügt dabei weiter über ein (auf der Speichereinheit abgelegtes) Berechnungsmodell, anhand welchem die Steuer- und Recheneinheit beispielsweise unter Berücksichtigung einer Konzentration des zumindest einen ausgewählten oder auswählbaren Blutanteilsstoffs im Blut des Patienten(körpers) oder einer Absorbanz (weil Konzentration zu Absorbanz äquivalent ist), vorzugsweise zu Beginn eines Behandlungszyklus mittels der Dialysemaschine, einen tatsächlichen Rezirkulationswert berechnet. Dafür schaltet die Steuer- und Recheneinheit zur Bestimmung des zumindest einen ausgewählten oder auswählbaren Blutanteilsstoffs im Blut des Patienten(körpers) die Dialysemaschine für die Dauer des im Datensatz angegebenen oder manuell (einschließlich Scann) eingegebenen Zeitwerts in den Kurzschlussmodus und betreibt den extrakorporalen Blutkreis, vorzugsweise gleichzeitig, bei dem angegebenen Blutstromwert und erfasst unmittelbar nach Beendigung des Kurzschlussmodus mithilfe der Sensorvorrichtung messtechnisch einen durch den Kurzschlussmodus entstandenen Konzentrationsbolus bzw. einen hierfür stehenden Messparameter in der aus dem Dialysator ablaufenden, verbrauchten Dialysierflüssigkeit.

In anderen Worten wird zur Bestimmung einer tatsächlichen Rezirkulation während einer Blutbehandlung zunächst die Dialysemaschine temporär in einem Bypassmodus betrieben. In diesem Bypassmodus wird die Dialysierflüssigkeitsseite im Dialysator nicht mit frischer Dialysierflüssigkeit versorgt bzw. die Filtermembran dialysierflüssigkeitsseitig nicht mit frischer Dialysierflüssigkeit umspült. Vielmehr wird die auf der Dialysierflüssigkeitsmembranseite vorhandene Dialysierflüssigkeit dort gehalten, vorzugsweise unter Einsatz von Ventilen, die in der Dialysierflüssigkeitszulaufleitung und der Dialysierflüssigkeitsablaufleitung angeordnet sind. Anstelle von Ventilen sind selbstverständlich auch andere zur Fluidabsperrung geeignete Elemente, beispielsweise Pumpen, denkbar. Während nun die auf der Dialysierflüssigkeitsmembranseite befindliche Dialysierflüssigkeit dort gehalten wird, wird der Blutstrom im extrakorporalen Blutkreis mit einer festgelegten Blutstromrate betrieben. Die Blutseite des Dialysators wird also weiter mit arteriellem bzw. zu reinigendem Blut versorgt bzw. die Filtermembran blutseitig weiter mit arteriellem bzw. zu reinigendem Blut umspült. Folglich findet nun, wie bei Dialysebehandlungen üblich, aufgrund von Diffusion ein Übertritt von im Blut gelösten Stoffen (Blutanteilsstoffen) durch die Filtermembran in die Dialysierflüssigkeit statt, und zwar solange, bis kein Konzentrationsgradient mehr zwischen dem auf der Blutseite vorhandenen Blut und der auf der Dialysierflüssigkeitsseite vorhandenen Dialysierflüssigkeit vorliegt, also in anderen Worten ein Diffusionsgleichgewicht erreicht ist. Da das Blut auf der Blutmembranseite des Dialysators weiter strömt, während die Dialysierflüssigkeit auf der Dialysierflüssigkeitsmembranseite des Dialysators steht, reichern sich in der stehenden Dialysierflüssigkeit solange Blutanteilsstoffe weiter an, bis diese in der stehenden Dialysierflüssigkeit zumindest im Wesentlichen dieselbe Konzentration erreicht haben wie im weiter durch den Dialysator strömenden Blut. In anderen Worten entspricht zu dem Zeitpunkt, an dem das Diffusionsgleichgewicht eines Blutanteilsstoffs zwischen der stehenden Dialysierflüssigkeit und dem strömenden Blut (im Wesentlichen) erreicht ist, die Konzentration des in der stehenden Dialysierflüssigkeit gelösten Blutanteilsstoffs zumindest im Wesentlichen der Konzentration des im Blut des Patientenkörpers gelösten Blutanteilsstoffs. An dieser Stelle sei darauf hingewiesen, dass der Parameter "Konzentration" auch durch einen mit diesem zusammenhängenden anderen Parameter repräsentiert sein kann wie beispielsweise eine Absorbance oder eine messbare elektrische Leitfähigkeit. Zur Berechnung der tatsächlichen Rezirkulation kann also mit Erreichen des Diffusionsgleichgewichts die in der stehenden Dialysierflüssigkeit vorliegende (direkt oder indirekt messbare/bestimmbare) Konzentration eines Blutanteilsstoffs ersatzweise für die im Blut des Patientenkörpers vorliegende Konzentration $c_{sys}$ dieses Blutanteilsstoffs hergenommen werden. Um eine etwaige Überschreitung des linearen Sensormessbereiches zu kompensieren, kann zusätzlich ein Kompensationsfaktor k genutzt werden. K kann hierbei durch eine Funktion ermittelt werden, die die Kennlinie des Sensors analytisch beschreibt.

Der Zeitpunkt, an dem ein Diffusionsgleichgewicht erreicht ist, hängt neben dem konkreten Blutanteilsstoff, beispielsweise ein bestimmtes urämisches Toxin, ggf. auch vom Wirkungsgrad der Blutbehandlung ab, also von der tatsächlich vorliegenden Rezirkulation. Ist die Rezirkulation nur gering ausgeprägt oder gar nicht vorhanden, wird das Diffusionsgleichgewicht ggf. schneller erreicht, als wenn die Rezirkulation hoch ausfällt, da rezirkuliertes Blut zu einer Verdünnung des zu reinigenden, arteriellen Bluts führt und somit die Effizienz der Blutreinigung beeinträchtigt. Die Dauer bis zum Erreichen des Diffusionsgleichgewichts zwischen der Blut- und der Dialysierflüssigkeitsseite ist maßgeblich aber abhängig vom eingestellten Blutfluss und der Dialysatorgröße. Je größer der Blutfluss und je kleiner der Dialysator, desto schneller wird ein diffusives Gleichgewicht erreicht.

Die Höhe der Rezirkulation hat dagegen einen Einfluss darauf, wie lange es dauert, bis die arterielle Blutkonzentration cBl der systemischen Konzentration cSYS entspricht. Im Falle einer nicht vorliegenden Rezirkulation entspricht cBl nahezu cSYS. Es ist daher davon auszugehen, dass dieser Vorgang weniger Zeit beansprucht als die oben erwähnte Herstellung des Diffusionsgleichgewichts.

Um nun sicherzustellen, dass die Dauer des Bypassmodus auch tatsächlich mindestens der Dauer, bis zu der sich das Diffusionsgleichgewicht eines bestimmten Blutanteilsstoffs im Dialysator eingestellt hat, entspricht, muss die Zeitdauer des Bypassmodus stets auf die Maximaldauer der Diffusionsgleichgewichtseinstellung ausgelegt werden. Diese wird der Steuer- und Recheneinheit vorab zur Verfügung gestellt, entweder durch Abruf der Information aus einer Datenbank / einem Datensatz oder durch Eingabe eines Benutzers, und kann abhängig vom Patienten, dessen Shuntzustand und/oder weiteren spezifischen Erfahrungswerten sein, beispielsweise empirische Ermittlung im Labor oder Auswertung von vergangenen Blutbehandlungen.

Die Dauer des Bypassmodus bzw. die zu erwartende Dauer bis zur Diffusionsgleichgewichtseinstellung im Dialysator ist weiter davon abhängig, was für ein Dialysator verwendet wird, beispielsweise vom Zustand (neu oder wiederverwendet) und/oder der (verfügbaren) Filterfläche und/oder des (dialysatseitigen) Füllvolumens, und mit welcher Strömungsrate das zur reinigende Blut durch den Dialysator geführt wird. Deshalb steht der Steuer- und Recheneinheit ein Datensatz zur Verfügung, aus dem sich die Soll-Dauer des Bypassmodus und die einzustellende Soll-Blutflussrate unter Berücksichtigung der maximal möglichen Rezirkulation und der Eigenschaften des konkret verwendeten / angeschlossenen Dialysators ermitteln lassen. Es ist alternativ auch denkbar, dass die Soll-Dauer des Bypassmodus auch in Abhängigkeit einer vorbestimmten Blutströmungsrate ermittelt wird.

Ist die Soll-Dauer des Bypassmodus dann erreicht und liegt folglich im Dialysator ein Diffusionsgleichgewicht für einen bestimmten Blutanteilsstoff zwischen Blutseite und Dialysierflüssigkeitsseite im Dialysator vor, wird der Bypassmodus beendet und werden somit die Ventile in Dialysierflüssigkeitszuführleitung und Dialysierflüssigkeitsablaufleitung wieder geöffnet. Mittels der Sensoreinrichtung, welche vorzugsweise stromab des Ventils in der Dialysierflüssigkeitsablaufleitung angeordnet ist, kann nun in der durch die Dialysierflüssigkeitsablaufleitung strömenden Dialysierflüssigkeit die Konzentration des bestimmten Blutanteilsstoffs bestimmt werden, welche der Konzentration des bestimmten Blutanteilsstoffs im Blut des Patientenkörpers entspricht.

Vorteilhafterweise kann somit eine tatsächlich vorliegende Rezirkulation bestimmt werden, ohne dass vorab durch separate Blutprobenentnahme am Patienten die Konzentration eines Blutanteilsstoffs im Blut des Patientenkörpers bestimmt werden muss, und folglich kann ein vollautomatisiertes, zeitsparendes und sicheres Blutbehandlungsverfahren ermöglicht werden.

Weiter bevorzugt ist die Speichereinheit in der Dialysemaschine fest integriert. Vorteilhafterweise muss somit nicht vor der Behandlung ein externes, zusätzliches Speichermedium, beispielsweise USB-Stick, angeschlossen werden.

## Kurzbeschreibung der Zeichnung

Die Erfindung wird nachstehend anhand eines bevorzugten Ausführungsbeispiels unter Bezugnahme auf die beigefügte Zeichnung näher beschrieben. Es zeigen:

Fig. 1 ein erstes Ausführungsbeispiel einer erfindungsgemäßen extrakorporalen Blutbehandlungsmaschine;

Fig. 2 ein zweites Ausführungsbeispiel einer erfindungsgemäßen extrakorporalen Blutbehandlungsmaschine;

Fig. 3 ein drittes Ausführungsbeispiel einer erfindungsgemäßen extrakorporalen Blutbehandlungsmaschine;

Fig. 4 einen zeitlichen Verlauf der Messaufnahme an einer Sensorvorrichtung einer Dialysemaschine, bei der die Sensorvorrichtung direkt hinter oder vor dem Ventil der Dialysierflüssigkeitsablaufleitung angeordnet ist.

Die Figuren sind lediglich schematischer Natur und dienen ausschließlich dem Verständnis der Erfindung. Die gleichen Elemente sind mit denselben Bezugszeichen bezeichnet.

**Detaillierte Beschreibung bevorzugter Ausführungsbeispiele**

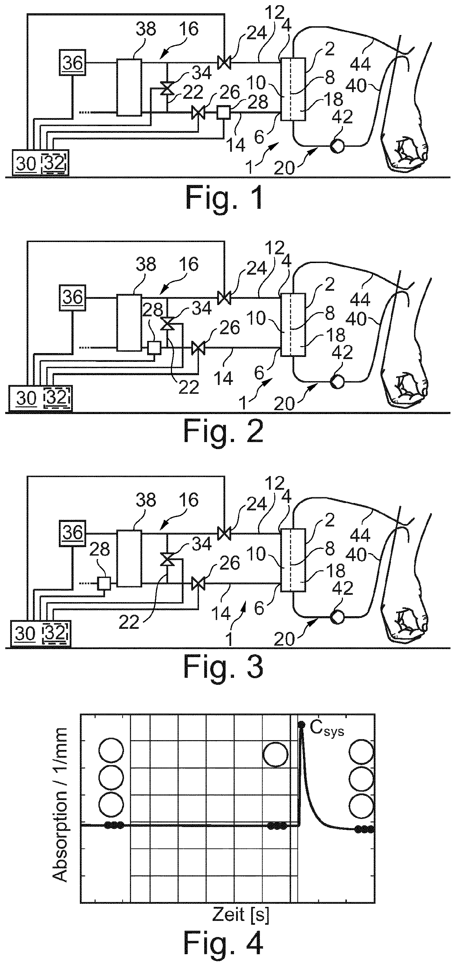

Fig. 1 zeigt ein erstes Ausführungsbeispiel einer erfindungsgemäßen extrakorporalen Blutbehandlungsmaschine (Dialysemaschine) 1 mit einem Dialysator 2, der einen Dialysierflüssigkeitszulauf 4 und einen Dialysierflüssigkeitsablauf 6 hat. Der Dialysator 2 ist weiter mit einer Filtermembran 8 ausgestattet, welche eine Dialysierflüssigkeitsmembranseite 10, die mit einer Dialysierflüssigkeitszulaufleitung 12 und einer Dialysierflüssigkeitsablaufleitung 14 eines Dialysierflüssigkeitskreises 16 in Fluidverbindung steht, von einer Blutmembranseite 18 trennt. Dabei sind die Dialysierflüssigkeitszulaufleitung 12 und die Dialysierflüssigkeitsablaufleitung 14 an den Dialysierflüssigkeitszulauf 4 und den Dialysierflüssigkeitsablauf 6 des Dialysators 2 angeschlossen. Die Blutmembranseite 18 steht in Fluidverbindung mit einem extrakorporalen Blutkreis 20. In dem Dialysierflüssigkeitskreis 16 ist weiter eine Bypassleitung 22 vorgesehen, mit der die Dialysierflüssigkeitsmembranseite 10 des Dialysators 2 überbrückt, also strömungstechnisch umgangen, werden kann. In der Dialysierflüssigkeitszulaufleitung 12 und der Dialysierflüssigkeitsablaufleitung 14 ist jeweils ein Ventil 24, 26 vorgesehen, mit denen die Dialysierflüssigkeitszulaufleitung 12 und die Dialysierflüssigkeitsablaufleitung 14 geöffnet bzw. geschlossen werden können und somit entweder den Strömungsweg für frische Dialysierflüssigkeit bei geöffneten Ventilen 24, 26 durch die Dialysierflüssigkeitszulaufleitung 12, die Dialysierflüssigkeitsmembranseite 10 des Dialysators 2 und die Dialysierflüssigkeitsablaufleitung 14 freigeben, oder durch Verschließen zumindest des Ventils 24, idealerweise beider Ventile 24, 26, diesen Strömungsweg verschließen.

In der ersten Ausführungsform der erfindungsgemäßen Dialysemaschine 1 befindet sich stromab des Dialysierflüssigkeitsablaufs 6 und stromauf des Ventils 26 eine Sensorvorrichtung 28. Diese ist beispielsweise als optische Sensorvorrichtung ausgebildet und nimmt in der vorbeiströmenden Dialysierflüssigkeit mittels UV/VIS-Spektroskopie abhängig vom Absorptionsbereich des konkreten zu messenden Blutanteilsstoffs Messungen bei der von diesem Blutanteilsstoff absorbierbaren Wellenlänge vor, um eine Konzentrationsbestimmung dieses Blutanteilsstoffs oder eine entsprechende Kenngrößenbestimmung zu ermöglichen. Konzentrations-/Kenngrößenbestimmungen durch Absorptionsmessung sind allgemein bekannt und werden daher an dieser Stelle nicht näher erläutert. Neben optischen Messverfahren sind auch alternative Messverfahren, beispielsweise durch Leitfähigkeitsbestimmung, denkbar. Die an der Sensorvorrichtung 28 generierten Messwerte werden an eine Steuer- und Recheneinheit 30 übertragen und von dieser ausgewertet.

Die Steuer- und Recheneinheit 30 steht zumindest mit den Ventilen 24, 26 und der Sensorvorrichtung 28 in gegenseitigem Informationsaustausch, d.h. sie empfängt und sendet Informationen von den Ventilen 24, 26 und der Sensorvorrichtung 28 bzw. an die Ventile 24, 26 und die Sensorvorrichtung 28. Weiter ist die Steuer- und Recheneinheit 30 mit einer Speichereinheit 32 verbunden oder ausgestattet. Auf dieser Speichereinheit 32 ist zumindest ein Datensatz abgelegt bzw. ablegbar, mit dem die Dialysemaschine 1 abhängig von den vorgegebenen bzw. empfangenen Informationen gesteuert werden kann.

Die Dialysemaschine 1 verfügt weiter über ein weiteres Ventil 34 in der Bypassleitung 22, das ebenfalls mit der Steuer- und Recheneinheit 30 in Informationsaustauschkontakt steht und von dieser gesteuert, also geöffnet und geschlossen werden kann. Das Ventil 34 ist vorgesehen, um den Strömungsweg für frische Dialysierflüssigkeit über die Bypassleitung 22 freizugeben oder zu sperren. Die frische Dialysierflüssigkeit wird aus einer Dialysierflüssigkeitsbereitstellungseinheit / Dialysierflüssigkeitsquelle 36 bezogen. Stromab der Dialysierflüssigkeitsquelle 36 befindet sich eine Bilanzkammer 38, die in den Dialysierflüssigkeitskreis 16 einströmende frische Dialysierflüssigkeit und aus diesem ausströmende (verbrauchte) Dialysierflüssigkeit bilanziert. Die Bilanzkammer 38 ist strömungstechnisch so angeordnet, dass diese sich zwischen der Dialysierflüssigkeitsquelle 36 und einer Mündungsstelle der Bypassleitung 22 in die Dialysierflüssigkeitszulaufleitung 12 sowie zwischen einer Mündungsstelle der Bypassleitung 22 in die Dialysierflüssigkeitsablaufleitung 14 und einem Abguss befindet.

Auf der Blutmembranseite 18 des Dialysators 2 ist der extrakorporale Blutkreis 20 an den Dialysator 2 angeschlossen. Der Blutkreis 20 weist zumindest eine arterielle Blutleitung 40 auf, die einen arteriellen Patientenzugang mit einem blutseitigen Dialysatoreingang verbindet und an der eine Blutpumpe 42 angeordnet ist, und eine venöse Blutleitung 44, die einen blutseitigen Dialysatorausgang mit einem venösen Patientenzugang verbindet. Die Blutpumpe 42 befindet sich ebenfalls in (gegenseitigem) Informationsaustauschkontakt mit der Steuer- und Recheneinheit 30 und wird von dieser gesteuert.

Fig. 2 zeigt ein zweites Ausführungsbeispiel einer erfindungsgemäßen Dialysemaschine 1, die sich von der Dialysemaschine 1 des ersten Ausführungsbeispiels lediglich darin unterscheidet, dass die Sensorvorrichtung 28 nicht stromauf des Ventils 26 angeordnet ist, sondern sich strömungstechnisch zwischen dem Ventil 26 und der Bilanzkammer 38, insbesondere zwischen der Mündungsstelle der Bypassleitung 22 in die Dialysierflüssigkeitsablaufleitung 14 und der Bilanzkammer 38, befindet.

Fig. 3 zeigt ein drittes Ausführungsbeispiel einer erfindungsgemäßen Dialysemaschine 1, die sich von der Dialysemaschine 1 des ersten Ausführungsbeispiels lediglich darin unterscheidet, dass die Sensorvorrichtung 28 nicht stromauf des Ventils 26 angeordnet ist, sondern sich stromab der Bilanzkammer 38 befindet.

Im Betrieb der Dialysemaschine 1 (alle Ausführungsbeispiele) wird zunächst ein Patient mit dem extrakorporalen Blutkreis 20 verbunden. Anschließend ruft die Steuer- und Recheneinheit 30 aus dem Datensatz, der auf der Speichereinheit 32 abgelegt ist, einen Soll-Blutstromwert und einen Zeitwert für die Dauer eines Bypassmodus ab. Dabei können patientenabhängige minimale und maximale Obergrenzen für den Soll-Blutstromwert im Datensatz Berücksichtigung finden. Der Zeitwert für die Dauer des Bypassmodus ist dabei vorzugsweise so gewählt/festgelegt, dass während des Bypassmodus auch unter dem schlechtesten Umstand, nämlich einer maximal möglichen oder maximal zu erwartenden Rezirkulation (z.B. Rezirkulation von 20% bis 30%), ein (nahezu) Diffusionsgleichgewicht für den zu messenden Blutanteilsstoff im Dialysator 2 erreicht wird. Hier gilt die Regel, dass je geringer der Blutstromwert und je größer der Dialysator ist, desto länger dauert es bis zum Erreichen des (nahezu) Diffusionsgleichgewichts. Abhängig von dem konkreten zu messenden/zu erfassenden Blutanteilsstoff, der optional vor der Behandlung aus mehreren möglichen Blutanteilsstoffen ausgewählt wird, kann der ausgegebene Zeitwert zusätzlich größer oder kleiner ausfallen.

Nachdem die Steuer- und Recheneinheit 30 den Soll-Zeitwert und den Soll-Blutstromwert abgerufen hat bzw. diese manuell eingegeben wurden, stellt sie die Blutpumpe 42 auf den Soll-Blutstromwert ein. Ist der Soll-Blutstromwert erreicht und ist auch ein vorgegebener Wert für den Dialysierflüssigkeitsstrom durch den Dialysierflüssigkeitskreis 16 erreicht, wird die Dialysemaschine 1 von der Steuer- und Recheneinheit 30 in den Bypassmodus geschaltet. Das bedeutet, dass für den Zeitraum des Bypassmodus die Ventile 24, 26 geschlossen sind, also die zwischen den Ventilen 24, 26 befindliche Dialysierflüssigkeit eingesperrt ist, und das Ventil 34 in der Bypassleitung 22 geöffnet ist, sodass der Strömungsweg der frischen Dialysierflüssigkeit aus der Dialysierflüssigkeitsquelle 36 über die Bypassleitung 22 zum Abguss führt. Für die Dauer des Bypassmodus wird nun die Blutpumpe 42 mit der eingestellten Rate betrieben, während die Dialysierflüssigkeit auf der Dialysierflüssigkeitsmembranseite 10 steht. Infolgedessen tritt aus dem die Blutmembranseite 18 des Dialysators 2 durchströmenden Blut der zu messende/bestimmende Blutanteilsstoff über die Filtermembran 8 in die auf der Dialysierflüssigkeitsmembranseite 10 des Dialysators 2 stehende Dialysierflüssigkeit über. Somit reichert sich der zu messende/bestimmende Blutanteilsstoff in der stehenden Dialysierflüssigkeit solange an, bis ein Diffusionsgleichgewicht auf der Blutmembranseite 18 und der Dialysierflüssigkeitsmembranseite 10 (im Wesentlichen) erreicht ist. Da das Blut im extrakorporalen Blutkreis 20 kontinuierlich weiter strömt, entspricht die Diffusionsgleichgewichtskonzentration des zu messenden/bestimmenden Blutanteilsstoffs in der stehenden Dialysierflüssigkeit zu diesem Zeitpunkt im Wesentlichen der im Blut des extrakorporalen Blutkreises 20 vorliegenden Konzentration des zu messenden/bestimmenden Blutanteilsstoffs, welche zu diesem Zeitpunkt wiederum der im Blut des Patientenkörpers vorliegenden Konzentration des zu messenden Blutanteilsstoffs entspricht. Folglich entspricht also die Diffusionsgleichgewichtskonzentration des zu messenden/bestimmenden Blutanteilsstoffs in der stehenden Dialysierflüssigkeit der im Blut des Patientenkörpers vorliegenden Konzentration des zu messenden Blutanteilsstoffs.

Ist das Diffusionsgleichgewicht (nahezu) erreicht, bzw. wurde der vorgegebene Zeitwert für den Bypassmodus, innerhalb dessen das Diffusionsgleichgewicht als erreicht gilt, erreicht, hebt die Steuer- und Recheneinheit 30 den Bypassmodus auf. Folglich schließt sie das Ventil 34 in der Bypassleitung 22 und öffnet zeitgleich die Ventile 24, 26 in der Dialysierflüssigkeitszulaufleitung 12 und der Dialysierflüssigkeitsablaufleitung 14, sodass frische Dialysierflüssigkeit von der Dialysierflüssigkeitsquelle 36 wieder durch die Dialysierflüssigkeitsmembranseite 10 des Dialysators 2 fließt. Die vorher auf der Dialysierflüssigkeitsmembranseite 10 gestandene Dialysierflüssigkeit fließt folglich durch die Dialysierflüssigkeitsablaufleitung 14 in Richtung Abguss, wobei sie die Sensoreinrichtung 28 passiert. Diese misst in Folge der Anreicherung des zu messenden Blutanteilsstoffs einen Peak (Lichtabsorptionspeak stellvertretend für die Konzentration dieses Blutanteilsstoffs), dessen Maximum als Diffusionsgleichgewichtskonzentration des Blutanteilsstoffs gewertet werden kann. Im Rückschluss ist also nach der Messung des Peakmaximums die im Blut des Patientenkörpers vorliegende Konzentration des zu messenden/bestimmenden Blutanteilsstoffs bekannt und bei der nachfolgenden bzw. fortgesetzten Blutbehandlung kann auf bekanntem Weg die tatsächliche Rezirkulation berechnet werden.

Fig. 4 zeigt den zeitlichen Verlauf der Messaufnahme (Absorptionswert des zu messenden Blutanteilsstoffs) an einer Sensorvorrichtung 28 einer Dialysemaschine 1, bei der die Sensorvorrichtung 28 direkt hinter dem Ventil 26 der Dialysierflüssigkeitsablaufleitung 14 und vor oder hinter der Mündungsstelle der Bypassleitung 22 in die Dialysierflüssigkeitsablaufleitung 14 angeordnet ist (der zeitliche Versatz des Peak bei einer Verschiebung der Sensorvorrichtung 28 in Richtung stromab ist in der Fig. 4 der Einfachheit halber als vernachlässigbar unberücksichtigt). Dabei ist der karriert hinterlegte Bereich der Zeitraum, in dem der Bypassmodus aktiv ist, also an der Sensorvorrichtung 28 keine von der Dialysierflüssigkeitsmembranseite 10 her strömende Dialysierflüssigkeit vorbeifließt. Vor und während des Bypassmodus misst die Sensorvorrichtung 28 in der Dialysierflüssigkeit folglich für einen bestimmten Blutanteilsstoff eine konstante Kenngröße für die Konzentration, da die Dialysierflüssigkeit bzw. die Dialysierflüssigkeitsfraktion, in der sich infolge von Diffusion der zu messende Blutanteilsstoff anreichert, zwischen den Ventilen 24, 26 eingesperrt ist und frische Dialysierflüssigkeit von der Dialysierflüssigkeitsquelle 36 die Sensorvorrichtung nicht umströmt. Die so gemessene Konzentration kann als die Konzentration des zu messenden Blutanteilsstoffs am Dialysatorausgang unter den normalen, bekannten Behandlungs-/Betriebsbedingungen angesehen werden ($c_{do}$).

Nach Beendigung des Bypassmodus misst die Sensorvorrichtung einen deutlichen Peak in der Konzentration des zu messenden Blutanteilsstoffs, da nach Öffnen des Ventils 26 die vorher eingesperrte Dialysierflüssigkeitsfraktion nun an der Sensorvorrichtung 28 vorbeiströmt. Hierbei ist anzumerken, dass für den Fall, in dem die Sensorvorrichtung 28 unmittelbar hinter dem Ventil 26 angeordnet ist, das Peakmaximum nahezu der Diffusionsgleichgewichtskonzentration entspricht. Befindet sich zwischen dem Ventil 26 und der Sensorvorrichtung 28 ein größerer Abstand, verringert sich in Folge von Diffusion das Peakmaximum, sodass entsprechende mathematische Korrekturmaßnahmen zur Bestimmung der Diffusionsgleichgewichtskonzentration herangezogen werden. Die gemessene / bestimmte Diffusionsgleichgewichtskonzentration des zu messenden Blutanteilsstoffs entspricht der systemischen Blutanteilsstoffkonzentration $c_{sys}$.

Hat der Konzentrationsbolus des zu messenden/bestimmenden Blutanteilsstoffs in der Dialysierflüssigkeit die Sensorvorrichtung 28 vollständig passiert, entspricht die gemessene/bestimmte Konzentration des Blutanteilsstoffs wieder $c_{do}$.

Mit der bekannten Gleichung (5) gemäß vorstehender Beschreibung kann nun die Rezirkulation R berechnet werden.


Liste der Bezugszeichen


1    extrakorporale Blutbehandlungsmaschine
2    Dialysator
4    Dialysierflüssigkeitszulauf
6    Dialysierflüssigkeitsablauf
8    Filtermembran
10    Dialysierflüssigkeitsmembranseite
12    Dialysierflüssigkeitszulaufleitung
14    Dialysierflüssigkeitsablaufleitung
16    Dialysierflüssigkeitskreis
18    Blutmembranseite
20    Blutkreis
22    Bypassleitung
24    Sperrventil
26    Sperrventil
28    Sensorvorrichtung
30    Steuer- und Recheneinheit
32    Speichereinheit
34    Ventil
36    Dialysierflüssigkeitsquelle
38    Bilanzkammer
40    arterielle Blutleitung
42    Blutpumpe
44    venöse Blutleitung


**Patentansprüche**

1.    Extrakorporale Blutbehandlungsmaschine (1), insbesondere Dialysemaschine, mit einem Dialysator (2) sowie einer Sensorvorrichtung (28), die auf einer Dialysierflüssigkeitsseite dem Dialysator (2) nachgeschaltet und mit einer Steuer- und Recheneinheit (30) elektrisch verbunden ist, die dafür vorgesehen und ausgebildet ist, auf Basis der Messsignale der Sensorvorrichtung (28) zumindest einen vorzugsweise ausgewählten oder auswählbaren Blutanteilsstoff in der verbrauchten Dialysierflüssigkeit sowohl qualitativ wie auch quantitativ zu bestimmen, **dadurch gekennzeichnet, dass** die Steuer- und Recheneinheit (30) ferner dafür vorgesehen und angepasst ist,

- die extrakorporale Blutbehandlungsmaschine in einen Modus zu schalten, in welchem eine Dialysierflüssigkeitsmenge im Dialysator (2) zumindest solange eingesperrt wird, bis sich auf der Dialysierflüssigkeitseite und der Blutseite des Dialysators (2) ein sich nicht mehr oder nur noch unwesentlich veränderndes Konzentrationsgleichgewicht des Blutanteilsstoffs einstellt, wobei ein Zeitraum, innerhalb welchen sich das Konzentrationsgleichgewicht einstellt, durch einen, vorzugsweise analytisch bestimmten, Zeitwert festgelegt ist und daraufhin
- in einen Modus zu wechseln, in welchem die Dialysierflüssigkeitsströmung aus dem Dialysator (2) zugelassen ist, um die zuvor eingesperrte Dialysierflüssigkeitsmenge als ein Dialysierflüssigkeitsbolus der Sensorvorrichtung (28) zur Bestimmung der darin enthaltenden Blutanteilsstoff-Konzentration zuzuführen, und
- aus der vorab bestimmten Blutanteilsstoff-Konzentration sowie einer Anzahl weiterer Maschinen- und/oder Einstellungsparameter einen aktuellen Rezirkulationswert zu berechnen.

2. Extrakorporale Blutbehandlungsmaschine (1) nach Anspruch 1, **dadurch gekennzeichnet, dass** die zu bestimmende Blutanteilsstoff-Konzentration dem maximalen Peak im Sensorsignal der Sensorvorrichtung (28) unmittelbar nach der Freigabe des Dialysierflüssigkeitsbolus aus dem Dialysator (2) entspricht.

3. Extrakorporale Blutbehandlungsmaschine (1) nach Anspruch 1 oder 2, **dadurch gekennzeichnet, dass** die Berechnung des aktuellen Rezirkulationswerts anhand eines Berechnungsmodells erfolgt, welches durch die folgende Formel definiert ist:

$$R = \frac{1 - \dfrac{c_{DO} \cdot Q_D}{c_{sys} \cdot K_D}}{1 - \dfrac{c_{DO} \cdot Q_D}{c_{sys} \cdot K_D} + \dfrac{c_{DO} \cdot Q_D}{c_{sys} \cdot Q_B}}$$

mit

| R | Rezirkulationsrate |
|---|---|
| cDO | Konzentration eines Stoffes am Dialysatausgang |
| csys | c_sys systemische Konzentration eines Blutanteilsstoffes, welche nicht rezirkulationsbehaftet ist. (Konzentration im Patienten) |
| QD | Dialysierflüssigkeitsflussrate |
| QB | Blutflussrate im extrakorporalen Blutschlauchsystem |
| KD | theoretische Clearance des Dialysators |
| KOA | dialysatorspezifisch |

4. Extrakorporale Blutbehandlungsmaschine (1) nach einem der vorstehenden Ansprüche, **dadurch gekennzeichnet, dass**

- der Dialysator (2) einen Dialysierflüssigkeitszulauf (4) für frische Dialysierflüssigkeit, einen Dialysierflüssigkeitsablauf (6) für verbrauchte Dialysierflüssigkeit und eine Filtermembran (8) aufweist, die eine Dialysierflüssigkeitsmembranseite (10), auf der der Dialysator (2) über eine Dialysierflüssigkeitszulaufleitung (12) und eine Dialysierflüssigkeitsablaufleitung (14) an einen Dialysierflüssigkeitskreis (16) angeschlossen ist, von einer Blutmembranseite (18) trennt, auf der der Dialysator (2) an einen extrakorporaler Blutkreis (20) angeschlossen oder anschließbar ist;
- eine Bypassleitung (22) vorgesehen ist, mittels derer die Dialysierflüssigkeitsmembranseite (10) wahlweise in einem Kurzschlussmodus überbrückbar ist, um im Dialysator (2) befindliche Dialysierflüssigkeit temporär einzusperren, wofür an der Dialysierflüssigkeitszulaufleitung (12) und der Dialysierflüssigkeitsablaufleitung (14) wenigstens jeweils ein Sperrventil (24, 26) zwischen der Bypassleitung (22) und dem Dialysator (2) angeordnet ist;
- die Steuer- und Recheneinheit (30) zur Steuerung der extrakorporalen Blutbehandlungsmaschine (1) mit einer Speichereinheit (32) ausgebildet oder ausrüstbar ist,

wobei die extrakorporale Blutbehandlungsmaschine (1) weiter aufweist:

- einen auf der Speichereinheit (32) abgelegten oder ablegbaren Datensatz, der eine Anzahl von für unterschiedliche Parameter der Blutbehandlungsmaschine geeignete Blutstromwerte im extrakorporalen Blutkreis (20) und zugehörige, vorzugsweise analytisch bestimmte, Zeitwerte angibt, innerhalb welchem im Dialysator (2) bei entsprechend eingestelltem Blutstromwert unter der Annahme eines maximal möglichen Rezirkulationswerts ein Konzentrationsausgleich zumindest eines ausgewählten oder auswählbaren Blutanteilsstoffs zwischen Blut im extrakorporalen Blutkreis (20) und im Dialysator (2) eingesperrter Dialysierflüssigkeit ausschließlich infolge von Diffusion beendet ist; und

- das vorzugsweise auf der Speichereinheit (32) abgelegte Berechnungsmodell, anhand welchem die Steuer- und Recheneinheit (30) unter Berücksichtigung einer Konzentration des zumindest einen ausgewählten oder auswählbaren Blutanteilsstoffs im Blut des Patienten, vorzugsweise vor dem Start oder zu Beginn eines Behandlungszyklus mittels der extrakorporalen Blutbehandlungsmaschine (1), einen tatsächlichen Rezirkulationswert berechnet, wofür die Steuer- und Recheneinheit (30) zur Bestimmung des zumindest einen ausgewählten oder auswählbaren Blutanteilsstoffs im Blut des Patienten die extrakorporale Blutbehandlungsmaschine (1) für die Dauer des im Datensatz angegeben Zeitwerts in den Kurzschlussmodus schaltet und den extrakorporalen Blutkreis (20), vorzugsweise gleichzeitig, bei dem angegeben Blutstromwert betreibt und unmittelbar nach Beendigung des Kurzschlussmodus einen durch den Kurzschlussmodus entstandenen Konzentrationsbolus in der aus dem Dialysator (2) ablaufenden, verbrauchten Dialysierflüssigkeit mithilfe der Sensorvorrichtung (28) messtechnisch erfasst, wobei vorzugsweise eine Einrichtung zur Identifizierung des Dialysators (2) und/oder zur Eingabe der Dialysatoridentifikation oder individueller Dialysatorparameter vorgesehen ist.

5. Extrakorporale Blutbehandlungsmaschine (1) nach Anspruch 1 oder 2, **dadurch gekennzeichnet, dass** die Speichereinheit (32) in der extrakorporalen Blutbehandlungsmaschine (1) fest integriert ist.

6. Maschinenlesbare Speichereinheit, auf welcher ein Verfahren zur Überwachung einer Rezirkulationsrate bei einer extrakorporalen Blutbehandlung unter Verwendung einer extrakorporalen Blutbehandlungsmaschine nach einem der vorstehenden Ansprüche mit den folgenden Überwachungsschritten gespeichert ist:

   - Festlegen eines Zeitwerts in Abhängigkeit eines eingestellten Blutstromwerts innerhalb welchem sich ein Konzentrationsgleichgewicht eines vorzugsweise zuvor ausgewählten Blutanteilsstoffs, vorzugsweise Harnstoff, einstellt
   - Betreiben der extrakorporalen Blutbehandlungsmaschine in einem Bypassmodus, in welchem unter Beibehaltung des Blutstromwerts eine Dialysierflüssigkeit in einem Dialysator (2) für den festgelegten Zeitwert eingesperrt ist,
   - Umschalten in einen Freigabemodus, in welchem die eingesperrte Dialysierflüssigkeit in Richtung Dialysatorauslass freigeben wird,
   - Erfassen eines Messpeaks im Rahmen einer sensorischen Messung eines Blutanteilsstoff-äquivalenten Messparameters in der eingesperrten Dialysierflüssigkeit nach Umschalten in den Freigabemodus
   - Vorzugsweise Identifizieren des Dialysators und
   - Berechnen der Rezirkulationsrate anhand eines Berechnungsmodells.

7. Maschinenlesbare Speichereinheit nach Anspruch 7 **dadurch gekennzeichnet, dass** das Berechnen der Rezirkulationsrate anhand des Berechnungsmodells erfolgt, welches durch die folgende Formel definiert ist:

$$R = \frac{1 - \dfrac{c_{DO} \cdot Q_D}{c_{sys} \cdot K_D}}{1 - \dfrac{c_{DO} \cdot Q_D}{c_{sys} \cdot K_D} + \dfrac{c_{DO} \cdot Q_D}{c_{sys} \cdot Q_B}}$$

mit

| R | Rezirkulationsrate |
|---|---|
| cDO | Konzentration eines Stoffes am Dialysatausgang |
| csys | c_sys systemische Konzentration eines Blutanteilsstoffes, welche nicht rezirkulationsbehaftet ist. (Konzentration im Patienten) |
| QD | Dialysierflüssigkeitsflussrate |
| QB | Blutflussrate im extrakorporalen Blutschlauchsystem |
| KD | theoretische Clearance des Dialysators |
| KOA | dialysatorspezifisch |

**Claims**

1. An extracorporeal blood treatment machine (1), especially a dialysis machine, comprising a dialyzer (2) and a sensor device (28) that is downstream of the dialyzer (2) on a dialysis fluid side and is electrically connected to a control and computing unit (30), which is provided and configured to both qualitatively and quantitatively determine, on the basis of measurement signals of the sensor device (28), at least one preferably selected or selectable blood component in the used dialysis fluid, **characterized in that** the control and computing unit (30) is further provided and adapted

   - to switch the extracorporeal blood treatment machine into a mode in which a dialysis fluid amount is confined within the dialyzer (2) at least until a concentration equilibrium of the blood component on the dialysis fluid side and the blood side of the dialyzer (2) is established that no longer changes, or changes insignificantly, wherein a period of time within which the concentration equilibrium is established is determined by a preferably analytically determined time value and thereupon
   - to change into a mode in which the dialysis fluid flow from the dialyzer (2) is permitted in order to feed the previously confined dialysis fluid amount as a dialysis fluid bolus to the sensor device (28) for determining the blood component concentration contained therein, and
   - to calculate a current recirculation value from the previously determined blood component concentration and from a number of further machine and/or adjustment parameters.

2. The extracorporeal blood treatment machine (1) according to claim 1, **characterized in that** the blood component concentration to be determined corresponds to the maximum peak in the sensor signal of the sensor device (28) directly after the release of the dialysis fluid bolus from the dialyzer (2).

3. The extracorporeal blood treatment machine (1) according to claim 1 or 2, **characterized in that** the calculation of the current recirculation value takes place by means of a calculation model defined by the following formula:

$$R = \frac{1 - \frac{c_{DO} \cdot Q_D}{c_{sys} \cdot K_D}}{1 - \frac{c_{DO} \cdot Q_D}{c_{sys} \cdot K_D} + \frac{c_{DO} \cdot Q_D}{c_{sys} \cdot Q_B}}$$

with

| | |
|---|---|
| R | recirculation rate |
| cDO | concentration of a substance at the dialysate output |
| csys | c_sys systemic concentration of a blood component which is not affected by recirculation (concentration in the patient) |
| QD | dialysis fluid flow rate |
| QB | blood flow rate in the extracorporeal blood line system |
| KD | theoretical clearance of the dialyzer |
| KOA | dialyzer-specific |

4. The extracorporeal blood treatment machine (1) according to any of the preceding claims, **characterized in that**

   - the dialyzer (2) comprises a dialysis fluid inlet (4) for fresh dialysis fluid, a dialysis fluid outlet (6) for used dialysis fluid, and a filter membrane (8) which separates a dialysis fluid membrane side (10), at which the dialyzer (2) is connected to a dialysis fluid circulation (16) via a dialysis fluid inlet line (12) and a dialysis fluid outlet line (14), from a blood membrane side (18) at which the dialyzer (2) is connected or connectable to an extracorporeal blood circulation (20);
   - a bypass line (22) is provided by means of which the dialysis fluid membrane side (10) is optionally bypassable in a bypass mode so as to temporarily confine dialysis fluid present in the dialyzer (2), for which purpose at least one respective check valve (24, 26) is provided at the dialysis fluid inlet line (12) and the dialysis fluid outlet line (14) between the bypass line (22) and the dialyzer (2);
- the control and computing unit (30) for controlling the extracorporeal blood treatment machine (1) is configured or adapted to be equipped with a memory unit (32),

wherein the extracorporeal blood treatment machine (1) further comprises:

- a data set stored or storable on the memory unit (32) and indicating a number of blood flow values suited for different parameters of the blood treatment machine in the extracorporeal blood circulation (20) and corresponding, preferably analytically determined, time values within which, in the dialyzer (2), with an appropriately adjusted blood flow value, assuming a maximally possible recirculation value, a concentration equilibrium of at least one selected or selectable blood component between blood in the extracorporeal blood circulation (20) and dialysis fluid confined in the dialyzer (2) is completed exclusively due to diffusion; and
- the calculation model preferably stored on the memory unit (32), by means of which the control and computing unit (30), taking account of a concentration of the at least one selected or selectable blood component in the blood of the patient, preferably prior to the start or at the beginning of a treatment cycle by means of the extracorporeal treatment machine (1), calculates an actual recirculation value, for which purpose the control and computing unit (30), for determination of the at least one selected or selectable blood component in the blood of the patient, switches the extracorporeal treatment machine (1) into the bypass mode for the duration of the time value indicated in the data set and operates the extracorporeal blood circulation (20), preferably simultaneously, at the blood flow value indicated and, directly after the termination of the bypass mode, metrologically determines, by means of the sensor device (28), a concentration bolus produced by the bypass mode in the used dialysis fluid draining from the dialyzer (2), wherein preferably a device for identifying the dialyzer (2) and/or for inputting the dialyzer identification or individual dialyzer parameters is provided.

5. The extracorporeal blood treatment machine (1) according to claim 1 or 2, **characterized in that** the memory unit (32) is steadily integrated in the extracorporeal blood treatment machine (1).

6. A machine-readable memory unit on which a method for monitoring a recirculation rate in an extracorporeal blood treatment by using an extracorporeal blood treatment machine in accordance with any of the preceding claims, comprising the following monitoring steps:

- determining a time value as a function of an adjusted blood flow value within which a concentration equilibrium of a preferably previously selected blood component, preferably urea, occurs,
- operating the extracorporeal blood treatment machine in a bypass mode in which, by maintaining the blood flow value, a dialysis fluid is confined within a dialyzer (2) for the determined time value,
- switching to a release mode in which the confined dialysis fluid is released in the direction of the dialyzer outlet,
- capturing a measurement peak in the scope of a sensory measurement of a blood component-equivalent measurement parameter in the confined dialysis fluid after switching to the release mode,
- preferably identifying the dialyzer, and
- calculating the recirculation rate by means of a calculation model.

7. The machine-readable memory unit according to claim 7, **characterized in that** the calculation of a recirculation rate takes place by means of the calculation model defined by the following formula:

$$R = \frac{1 - \frac{c_{DO} \cdot Q_D}{c_{sys} \cdot K_D}}{1 - \frac{c_{DO} \cdot Q_D}{c_{sys} \cdot K_D} + \frac{c_{DO} \cdot Q_D}{c_{sys} \cdot Q_B}}$$

with

| R | recirculation rate |
|---|---|
| cDO | concentration of a substance at the dialysate output |

(continued)

| csys | c_sys systemic concentration of a blood component which is not affected by recirculation (concentration in the patient) |
|------|------|
| QD | dialysis fluid flow rate |
| QB | blood flow rate in the extracorporeal blood line system |
| KD | theoretical clearance of the dialyzer |
| K0A | dialyzer-specific |

**Revendications**

1. Machine de traitement extracorporel du sang (1), en particulier machine de dialyse, avec un dialyseur (2) ainsi qu'un dispositif de capteur (28) qui est monté en aval du dialyseur (2) sur un côté liquide de dialyse et relié électriquement à une unité de commande et de calcul (30) qui est prévue et conçue pour déterminer, sur la base des signaux de mesure du dispositif de capteur (28), au moins une fraction de sang de préférence sélectionnée ou sélectionnable dans le liquide de dialyse usé, aussi bien qualitativement que quantitativement, **caractérisée en ce que** l'unité de commande et de calcul (30) est en outre prévue et adaptée pour

   - commuter la machine de traitement extracorporel du sang dans un mode dans lequel une quantité de liquide de dialyse est emprisonnée dans le dialyseur (2) au moins jusqu'à ce que s'établisse, du côté liquide de dialyse et du côté sang du dialyseur (2), un équilibre de concentration de la fraction de sang ne se modifiant plus ou plus que de manière insignifiante, dans laquelle une période au cours de laquelle s'établit l'équilibre de concentration est fixée par une valeur de temps, de préférence déterminée de manière analytique, puis
   - basculer vers un mode dans lequel le flux de liquide de dialyse provenant du dialyseur (2) est autorisé à amener la quantité de liquide de dialyse préalablement emprisonnée sous forme de bolus de liquide de dialyse au dispositif de capteur (28) pour déterminer la concentration de fraction de sang qu'il contient, et
   - calculer une valeur de recirculation actuelle à partir de la concentration de fraction de sang préalablement déterminée ainsi que d'un certain nombre de paramètres de machine et/ou de réglage supplémentaires.

2. Machine de traitement extracorporel du sang (1) selon la revendication 1, **caractérisée en ce que** la concentration de fraction de sang à déterminer correspond au pic maximal dans le signal de capteur du dispositif de capteur (28) immédiatement après la libération du bolus de liquide de dialyse du dialyseur (2).

3. Machine de traitement extracorporel du sang (1) selon la revendication 1 ou 2, **caractérisée en ce que** le calcul de la valeur de recirculation actuelle est effectué à l'aide d'un modèle de calcul défini par la formule suivante :

$$R = \frac{1 - \dfrac{c_{DO} \cdot Q_D}{c_{sys} \cdot K_D}}{1 - \dfrac{c_{DO} \cdot Q_D}{c_{sys} \cdot K_D} + \dfrac{c_{DO} \cdot Q_D}{c_{sys} \cdot Q_B}}$$

avec

| R | Taux de recirculation |
|------|------|
| cDO | Concentration d'une substance à la sortie de dialysat |
| csys | c_sys Concentration systémique d'une fraction de sang, laquelle n'est pas soumise à une recirculation. (concentration chez le patient) |
| QD | Débit du liquide de dialyse |
| QB | Débit sanguin dans le circuit sanguin extracorporel |
| KD | Clairance théorique du dialyseur |

(suite)

| K0A | Spécifique au dialyseur |
|-----|-------------------------|

**4.** Machine de traitement extracorporel du sang (1) selon l'une quelconque des revendications précédentes, **caractérisée en ce que**

- le dialyseur (2) présente une arrivée de liquide de dialyse (4) pour du liquide de dialyse frais, une sortie de liquide de dialyse (6) pour du liquide de dialyse usé et une membrane filtrante (8) qui sépare un côté membrane de liquide de dialyse (10), sur lequel le dialyseur (2) est relié à un circuit de liquide de dialyse (16) par l'intermédiaire d'une conduite d'arrivée de liquide de dialyse (12) et une conduite de sortie de liquide de dialyse (14), d'un côté membrane sanguine (18), sur lequel le dialyseur (2) est relié ou peut être relié à un circuit sanguin extracorporel (20) ;
- une conduite de dérivation (22) est prévue au moyen de laquelle le côté membrane de liquide de dialyse (10) peut être ponté au choix dans un mode de court-circuit pour temporairement emprisonner le liquide de dialyse se trouvant dans le dialyseur (2), pour lequel au moins une vanne d'arrêt (24, 26) est disposée au niveau de la conduite d'arrivée de liquide de dialyse (12) et de la conduite de sortie de liquide de dialyse (14) entre la conduite de dérivation (22) et le dialyseur (2) ;
- l'unité de commande et de calcul (30) pour la commande de la machine de traitement extracorporel du sang (1) est conçue avec ou peut être équipée d'une unité de stockage (32),

dans laquelle la machine de traitement extracorporel du sang (1) présente en outre :

- un jeu de données stocké ou pouvant être stocké sur l'unité de stockage (32) qui indique un certain nombre de valeurs de flux sanguin dans le circuit sanguin extracorporel (20) adaptées pour différents paramètres de la machine de traitement du sang et des valeurs de temps correspondantes, de préférence déterminées de manière analytique, dans lequel, dans le dialyseur (2), pour une valeur de flux sanguin réglée de manière correspondante et en supposant une valeur de recirculation maximale possible, un équilibrage de concentration d'au moins une fraction de sang sélectionnée ou pouvant être sélectionnée entre le sang dans le circuit sanguin extracorporel (20) et le liquide de dialyse emprisonné dans le dialyseur (2) est terminé exclusivement à la suite de la diffusion ; et
- le modèle de calcul stocké de préférence sur l'unité de stockage (32), à l'aide duquel l'unité de commande et de calcul (30) calcule, en tenant compte d'une concentration de l'au moins une fraction de sang sélectionnée ou pouvant être sélectionnée dans le sang du patient, de préférence avant le démarrage ou au début d'un cycle de traitement au moyen de la machine de traitement extracorporel du sang (1), une valeur de recirculation effective, pour laquelle l'unité de commande et de calcul (30) pour la détermination de l'au moins une fraction de sang sélectionnée ou pouvant être sélectionnée dans le sang du patient commute la machine de traitement extracorporel du sang (1) en mode de court-circuit pendant la durée de la valeur de temps indiquée dans le jeu de données et fait fonctionner le circuit sanguin extracorporel (20), de préférence simultanément, à la valeur de flux sanguin indiquée et, immédiatement après la fin du mode de court-circuit, détecte par une technique de mesure, à l'aide du dispositif de détection (28), un bolus de concentration créé par le mode de court-circuit dans le liquide de dialyse usé s'écoulant du dialyseur (2), dans laquelle un appareil d'identification du dialyseur (2) et/ou de saisie de l'identification de dialyseur ou de paramètres individuels de dialyseur est de préférence prévu.

**5.** Machine de traitement extracorporel du sang (1) selon la revendication 1 ou 2, **caractérisée en ce que** l'unité de stockage (32) est intégrée de manière fixe dans la machine de traitement extracorporel du sang (1).

**6.** Unité de stockage lisible par machine sur laquelle est stocké un procédé de surveillance d'un taux de recirculation lors d'un traitement extracorporel du sang en utilisant une machine de traitement extracorporel du sang selon l'une quelconque des revendications précédentes avec les étapes de surveillance suivantes :

- détermination d'une valeur de temps en fonction d'une valeur de flux sanguin réglée, au cours de laquelle s'établit un équilibre de concentration d'une fraction de sang de préférence préalablement sélectionnée, de préférence l'urée
- fonctionnement de la machine de traitement extracorporel du sang dans un mode de dérivation dans lequel, tout en conservant la valeur de flux sanguin, un liquide de dialyse est emprisonné dans un dialyseur (2) pendant la valeur de temps fixée,

- commutation dans un mode de libération dans lequel le liquide de dialyse emprisonné est libéré en direction de la sortie de dialyseur,

- détection d'un pic de mesure dans le cadre d'une mesure sensorielle d'un paramètre de mesure équivalent à la fraction de sang dans le liquide de dialyse emprisonné après commutation dans le mode de libération

- de préférence identification du dialyseur et

- calcul du taux de recirculation à l'aide d'un modèle de calcul.

7. Unité de stockage lisible par machine selon la revendication 7, **caractérisée en ce que** le calcul du taux de recirculation est effectué à l'aide du modèle de calcul qui est défini par la formule suivante :

$$R = \frac{1 - \dfrac{c_{DO} \cdot Q_D}{c_{sys} \cdot K_D}}{1 - \dfrac{c_{DO} \cdot Q_D}{c_{sys} \cdot K_D} + \dfrac{c_{DO} \cdot Q_D}{c_{sys} \cdot Q_B}}$$

avec

| R | Taux de recirculation |
|---|---|
| cDO | Concentration d'une substance à la sortie de dialysat |
| csys | c_sys Concentration systémique d'une fraction de sang, laquelle n'est pas soumise à une recirculation. (concentration chez le patient) |
| QD | Débit du liquide de dialyse |
| QB | Débit sanguin dans le circuit sanguin extracorporel |
| KD | Clairance théorique du dialyseur |
| K0A | Spécifique au dialyseur |

Fig. 1

Fig. 2

Fig. 3

Fig. 4

**IN DER BESCHREIBUNG AUFGEFÜHRTE DOKUMENTE**

**In der Beschreibung aufgeführte Patentdokumente**

- DE 19702441 C1
- EP 2783715 A1
- US 5588959 A
- US 5849179 A
- WO 9608305 A1
- DE 102013103221 A1

**In der Beschreibung aufgeführte Nicht-Patentliteratur**

- **MICHAELS.** Operating Parameters and Perform-ance Criteria for Hemodialyzers and other Membrane - Separation Devices. *Trans Amer Soc Artif Intern Organs,* 1966, vol. 12, 387-392